# EUROPÄISCHE PATENTSCHRIFT

(11) **EP 0 609 471 B1**
(45) Veröffentlichungstag und Bekanntmachung des Hinweises auf die Patenterteilung: **05.01.2000**
(21) Anmeldenummer: 93101553.1
(22) Anmeldetag: 02.02.1993
(51) Int. Cl.: A61K 38/39

(54) **Verwendung von Kollagen zur Behandlung von krankhaften Gelenkprozessen**
Use of collagen for the treatment of diseased joint processes
Utilisation de collagène pour le traitement des processus articulaires malades

(43) Veröffentlichungstag der Anmeldung: 10.08.1994
(73) Patentinhaber: Klaus, Edwin, Dr., D-97070 Würzburg (DE)
(72) Erfinder: Klaus, Edwin, Dr., D-97070 Würzburg (DE)
(74) Vertreter: Grünecker, Kinkeldey, Stockmair & Schwanhäusser Anwaltssozietät

(56) Entgegenhaltungen:
- EP-A- 0 254 289
- WO-A-92/06708
- BE-A- 701 570
- CH-A- 494 250
- DE-A- 2 405 002
- DE-A- 2 462 222
- FR-A- 2 613 620
- US-A- 3 966 908

## Beschreibung

Die Erfindung betrifft die Verwendung von Kollagen zur Behandlung von krankhaften Gelenkprozessen, insbesondere von degenerativen Gelenkprozessen.

Der Grundtypus der degenerativen, nicht entzündlichen Gelenkerkrankungen ist die Arthropathia oder Arthrosis deformans des höheren Lebensalters. Befallen sind hauptsächlich breitwüchsige Menschen, mehr Männer als Frauen, nach dem 50. Lebensjahr. Das Leiden beginnt schleichend, verläuft aber auch in Schühen und zu Beginn schmerzlos. Die Gelenke sind nur bei bestimmten Bewegungen empfindlich. Schonung der Gelenke begünstigt das Fortschreiten des Leidens, doch Übung sowie Massagen bringen Abhilfe. Durch Behandlung mit Ultrakurzwellen können auch gute Erfolge erzielt werden.

Weitere bisher übliche Therapien sind die systematische Verabreichung von nicht steroidalen Antirheumatika und gegebenenfalls intraartikuläre Injektionen von Chondroprotektiva, Homöopathika, Lokalanästhetika bzw. Cortison. Diese Therapien haben jedoch den Nachteil, daß die durch die degenerativen Gelenkprozesse letztendlich eintretende Gelenkzerstörung nicht verhindert, sondern nur hinausgezögert werden kann, da die Neubildung von Gelenkschmierstoffen, wie beispielsweise Mucopolysaccharide, nicht angeregt werden kann und auch von außen kein Gelenkschmierstoff in das betroffene Gelenk eingebracht wird.

Rekonstituiertes Typ-II-Kollagen kann zur Detektion von Antikörpern gegen intaktes Typ-II-Kollagen im Blut bei Arthrorheumatismus verwendet werden (JP-A-62-012 800).

Kollagene sind langfaserige, linearkolloide, hochmolekulare Skleroproteine der extrazellulären Matrix, die in Bindegeweben (z.B. Haut, Knorpel, Sehnen, Bänder und Blutgefäße), in der eiweißhaltigen Grundsubstanz des Knochens (Ossein) und im Dentin (Zahnbein) zusammen mit Proteoglykanen vorkommen. Sie gelten mit einem Anteil von 25 bis 30% als die mengenmäßig häufigsten tierischen Proteine. Die strukturelle Grundeinheit des Kollagens, das Tropo-Kollagen (MG. ca. 300.000), besteht aus drei Polypeptid-Ketten in Form spezieller linksgewundener Helices, die - nach einer auch in der Seilerei bekannten Technik - wiederum rechtsgängig umeinander gedreht sind (Tripelhelix). Das Tropo-Kollagen-Molekül ist ein "Seil" von 280 nm Länge bei einem Durchmesser von 1,4 nm; die Fibrillen messen im Querschnitt 200-500 nm (siehe Römpps Chemielexikon, Bd. 3, S. 2297, 1990).

Im Gegensatz zu den meisten Proteinen des Tierkörpers werden die Kollagene nicht fortlaufend erneuert. Sie nehmen - einmal gebildet - nicht weiter am Stoffwechsel teil und altern durch regelmäßige Zunahme der Vernetzung infolge Bildung von Wasserstoff-Brückenbindungen, Ester-Bindungen von Aminosäure- mit Zucker-Resten und von Isopeptid-Bindungen zwischen langgestreckten Aminosäure-Ketten.

In der Medizin wird modifiziertes Kollagen als temporärer Hautersatz, als Ersatz für Hornhaut und Glaskörperflüssigkeit des Auges, Ersatz für Sehnen, Sehnenscheiden, Hohlorgane und Adern, als Blutplasma-Ersatzstoff und in der Wundheilung erprobt (EP-B 52288, JP-A-52-076 416). Kollagen-Schwamm wird u.a. als Blutstillungsmittel verwendet. Kollagen enthaltende Arzneimittel werden zur Verbesserung erweiterungsfähiger Deformationen der Haut verwendet, wie sie bei Narben, durch unterschiedliche Ursachen hervorgerufenem Hautschwund und altersbedingten Hautfalten auftreten. Diese kollagenhaltigen Arzneimittel werden unter die aufzufüllenden Hautstellen injiziert und enthalten aus diesem Grund in der Regel ein örtliches Betäubungsmittel.

EP-A-499164 betrifft eine Injektionssuspension, umfassend ein physiologisch verträgliches hydrophiles Polymer, wie Kollagen, suspendiert in einem nichttoxischem flüssigen hydrophoben Träger. Diese Suspension wird zur Behandlung von degenerativen Gelenkerkrankungen, wie Osteoarthrose (siehe Spalte 4, Zeilen 44 ff.), verwendet, indem sie in das betroffene Gelenk injiziert wird. In dem Gelenk diffundiert die nichttoxische hydrophobe Flüssigkeit von dem Polymer ab und wird durch wäßrige Körperflüssigkeiten ersetzt. Das in der Suspension enthaltene Polymer verbleibt im Gelenk und quillt durch Absorption der wäßrigen Körperflüssigkeiten auf und ersetzt so die fehlende Gelenkschmiere (siehe Spalte 7, Zeilen 2 bis 19). Bei den in EP-A-499164 verwendeten hydrophilen Polymeren handelt es sich somit um wasserlösliche Stoffe, beispielsweise wasserlösliches Kollagen.

Aufgabe der vorliegenden Erfindung ist es, ein Arzneimittel zur Therapie krankhafter, insbesondere degenerativer Gelenkprozesse herzustellen, das die oben genannten Nachteile nicht aufweist.

Die Aufgabe wird erfindungsgemäß gelöst durch die Verwendung von unlöslichem Kollagen zur Herstellung eines Arzneimittels zur Behandlung von degenerativen, nichtentzündlichen Gelenkprozessen, wobei das Kollagen in einer Injektionssuspension enthalten ist, mit der Maßgabe, daß die Injektionssuspension keinen flüssigen hydrophoben Träger enthält.

Bei dem verwendeten Kollagen handelt es sich um unlösliches Kollagen, das beispielsweise aus Kälber- oder Rinderhaut gewonnen werden kann; bevorzugt ist jedoch Kollagen aus Rindernaut.

Das Kollagen ist bevorzugt in einer Injektionssuspension, insbesondere bevorzugt in einer Menge von etwa 20 bis 50 mg/ml, enthalten. Die Injektionssuspension wird erfindungsgemäß bei degenerativen Gelenkerkranungen, wie Arthrose oder aktivierter Arthrose, durch Injektion in die Gelenke appliziert. Aus diesem Grund ist bevorzugt ein lokales Betäubungsmittel, wie Bupivacain oder Lidocain, enthalten. Insbesondere bevorzugt ist Lidocain in der Injektionssuspension enthalten. Die bevorzugte Menge des enthaltenen lokalen Betäubungsmittels beträgt 1-5 mg/ml.

Gegenüber den bisherigen Therapien hat die erfindungsgemäße intraartikuläre Verwendung von Kollagen in einem Arzneimittel den Vorteil, daß es den bei einer Arthrose nur noch unzulänglich stattfindenden Gleitvorgang durch seine Eigenschaften begünstigt, ohne irgendwelche Substanzen anzugreifen, d.h. ohne zu schaden.

Begleitend können "Trägersubstanzen", beispielsweise Chondroprotektiva, in das Kollagen eingearbeitet werden.

Beispiele für derartige Chondoprotektiva sind die Präparate Dona 200 (Wirkstoff: D-Glucosamin) und Dona 22S (Wirkstoff: D-Glucosaminsulfat), sowie die Homöopathika Zeel und Heel.

Das Homöopathikum Heel setzt sich wie folgt zusammen:
2,2 ml enthalten: Auszug (1:10) aus Cartilago suis 22 ng,
Auszug (1:10) aus Funiculus umbilicalis suis 22 ng,
Auszug (1:10) aus Embryo suis 22 ng,
Auszug (1:10) aus Placenta suis (hormonfrei) 22 ng,
Rhus toxicodendr. 0̸ 0,22 mg, Arnica 0̸ 0,22 mg, Dulcamara 0̸ 0,22 µg, Symphytum 0̸ 22 µg, Sanguinaria 0̸ 33 µg,
Sulfur 0̸ 39,6 µg, Nacid 0,22 ng, Coenzym A 0,22 ng, (±)-a-Liponsäure 0,22 ng, Natriumoxalacetat 0,22 ng.
Das Arzneimittel kann außerdem noch angefärbt werden, beispielsweise mit kompatiblen Farbstoffen.

Die nachstehenden Beispiele erläutern die Erfindung.

### Beispiele 1 bis 6

In den folgenden Beispielen sind verschiedene Zusammensetzungen der Wirkstoffkombination in der Injektionssuspension angegeben:

1 ml Injektionssuspension enthaltend:
- 1.: 20 mg Kollagen aus Kälberhaut
2 mg Bupivacain;
- 2.: 30 mg Kollagen aus Rinderhaut
2 mg Bupivacain;
- 3.: 35 mg Kollagen aus Rinderhaut
3 mg Lidocain;
- 4.: 40 mg Kollagen aus Rinderhaut
3 mg Lidocain;
- 5.: 50 mg Kollagen aus Kälberhaut
3,5 mg Bupivacain.

### Anwendungsbeispiel 1

Eine junge Frau, 32 Jahre alt, mit therapieresistenter Gonalgie, wurde mit vor etwa 1 1/2 Jahren mit Injektionen von 0,5 ml der kollagenhaltigen Injektionssuspension gemäß Beispiel 3 auf der einen und 1,0 ml auf der anderen Seite intraartikulär in die Gelenke über etwa 1 1/2 Jahre behandelt; sie war anschließend vollkommen beschwerdefrei. Eine Wiederholung der Therapie war nicht notwendig.

### Anwendungsbeispiel 2

Injektionen bei Extension des Hüftgelenkes ergaben nach Vorspritzen von 2 ml Lokalanästhesie, dies nur zum Zwecke der richtigen Nadellage, eine Besserung, nachdem 2 ml der kollagenhaltigen Injektionssuspension gemäß Beispiel 3 pro Gelenkseite intraartikulär infiltriert wurden. Der Beobachtungszeitraum betrug annähernd zwei Jahre. Es wurde festgestellt, daß vor allem die primären arthrotischen Anlaufbeschwerden nach einem halben Jahr vollkommen zum Stillstand gekommen waren.
Weitere ähnliche Kasuistiken liegen vor.

### Anwendungsbeispiel 3

Intraartikuläre Injektion von 1 ml der kollagenhaltigen Injektionssuspension gemäß Beispiel 3 erbrachte bei einer linksseitigen starken Gonarthrose nach Angabe der Patientin eine Besserung der Beschwerden von etwa 70 %. Die Beschwerden sind seit etwa 2 Jahren konstant geblieben.

## Patentansprüche

1. Verwendung von unlöslichem Kollagen zur Herstellung eines Arzneimittels zur Behandlung von degenerativen, nichtentzündlichen Gelenkprozessen, wobei das Kollagen in einer Injektionssuspension enthalten ist,
mit der Maßgabe, daß die Injektionssuspension keinen flüssigen hydrophoben Träger enthält.

2. Verwendung nach Anspruch 1, **dadurch gekennzeichnet**, daß die Menge des in der Injektionssuspension enthaltenen Kollagens etwa 20-50 mg/ml beträgt.

3. Verwendung der Ansprüche 1 oder 2, **dadurch gekennzeichnet**, daß weiterhin ein lokales Betäubungsmittel enthalten ist.

4. Verwendung nach Anspruch 3, **dadurch gekennzeichnet**, daß die Menge des in der Injektionssuspension enthaltenen lokalen Betäubungsmittels 1 - 5 mg/ml beträgt.

5. Verwendung nach einem der Ansprüche 1 bis 4, **dadurch gekennzeichnet**, daß das in dem Arzneimittel enthaltene Kollagen Kollagen aus Rinderhaut ist.

## Claims

1. Use of insoluble collagen for the production of a medicament for the treatment of degenerative non-inflammatory articular processes, the collagen being contained in an injection suspension, subject to the condition that the injection suspension contains no liquid hydrophobic carrier.

2. Use according to Claim 1, characterized in that the quantity of collagen contained in the injection suspension is of the order of 20-50 mg/ml.

3. Use according to Claim 1 or Claim 2, characterized in that a local anaesthetic is also contained.

4. Use according to Claim 3, characterized in that the quantity of local anaesthetic contained in the injection suspension is 1 - 5 mg/ml.

5. Use according to any one of Claims 1 to 4, characterized in that the collagen contained in the medicament is collagen derived from bovine skin.

## Revendications

1. Utilisation de collagène insoluble pour fabriquer un médicament pour le traitement de pathologies articulaires dégénératives et non inflammatoires, selon laquelle le collagène est contenu dans une suspension pour injection, sous réserve que la suspension pour injection ne contienne pas de support hydrophobe liquide.

2. Utilisation selon la revendication 1, **caractérisée** en ce que la quantité de collagène contenu dans la suspension pour injection est d'environ 20 à 50 mg/ml.

3. Utilisation selon la revendication 1 ou 2, **caractérisée** en ce qu'un anesthésique local est également contenu dans la suspension.

4. Utilisation selon la revendication 3, **caractérisée** en ce que la quantité d'anesthésique local contenu dans la suspension pour injection est de 1 à 5 mg/ml.

5. Utilisation selon l'une des revendications 1 à 4, **caractérisée** en ce que le collagène contenu dans le médicament est du collagène provenant de peau de boeuf.
